# EUROPÄISCHE PATENTANMELDUNG

(11) **EP 1 380 279 A1**
(43) Veröffentlichungstag der Anmeldung: **14.01.2004**
(21) Anmeldenummer: 03014420.8
(22) Anmeldetag: 30.06.2003
(51) Int. Cl.: A61K 7/00

(54) **Kosmetische oder dermatologische Formulierungen zur Pflege der Gesichtshaut**

(30) Priorität: 04.07.2002 DE 10230061
(71) Anmelder: Beiersdorf AG, 20253 Hamburg (DE)
(72) Erfinder: Bürger, Anette, 22301 Hamburg (DE); Raschke, Thomas, Dr., 25421 Pinneberg (DE); Nielsen, Jens, 24558 Henstedt-Ulzburg (DE); Schulz, Jens, Dr., 22869 Schenefeld (DE)

(57) **Zusammenfassung**

Kosmetische oder dermatologische O/W-Emulsionen zur Pflege der Gesichtshaut, enthaltend
A) mindestens einen Emulgator, gewählt aus der Gruppe der PEG-Ester und/oder PEG-Ether der folgenden Struktur:

   R¹-(CH₂-CH₂-O)ₙ-R²,

   worin R1 und R2 unabhängig voneinander gewählt werden aus der Gruppe: Wasserstoff, Alkyl- und Acylreste mit 12 bis 40 Kohlenstoffatomen und n eine ganze Zahl zwischen 30 und 100 darstellt,
B) mindestens eine UV-Filtersubstanz gewählt aus der Gruppe I, bestehend aus Phenylen-1,4-bis-(2-benzimidazyl)-3,3'-5,5'-tetrasulfonsäure und ihren Salzen
C) mindestens eine UV-Filtersubstanz gewählt aus der Gruppe II, bestehend aus 2-Phenylbenzimidazol-5-sulfonsäure und ihre Salzen und
D) mindestens eine UV-Filtersubstanz gewählt aus der Gruppe III, bestehend aus den Estern der Zimtsäure
und ihre Verwendung.

## Beschreibung

Die vorliegende Erfindung betrifft kosmetische oder dermatologische Formulierungen zur Pflege der Gesichtshaut, insbesondere kosmetische oder dermatologische Formulierungen, welche die Gesichtshaut gezielt pflegen und dabei gleichzeitig die Einwirkung von UV-Strahlung vermindern.

Neben den positiven Auswirkungen des Sonnenlichtes, wie dem allgemeinen Wohlbefinden, der Bildung von Vitamin D3 und der Aknebehandlung, gibt es auch negative Auswirkungen, denen es entgegenzuwirken gilt.

Setzt man die Haut zu lange der Sonne oder einer künstlichen Strahlenquelle aus, so entwickelt sich nach einer Latenzzeit von 2 bis 3 Stunden eine gegen die unbestrahlte Haut stark abgegrenzte Hautrötung, das Erythema solare. Bei dem so entstehenden Sonnenbrand unterscheidet man zwischen
■ 1. Grad: Erythem (Rötung, Wärmegefühl)
   klingt nach 2 bis 3 Tagen wieder ab und verschwindet unter gleichzeitig zunehmender Pigmentierung,
■ 2. Grad: Blasenbildung
   auf der Haut bilden sich Blasen mit Brennen und Jucken, die Oberhaut wird flächig abgestoßen
■ 3. Grad: Zellschädigung
   es treten tiefgehende Zellschädigungen auf, der Körper reagiert mit Fieber, die Oberhaut wird großflächig abgestoßen.
Der 2. und 3. Grad werden auch als Dermatitis solare bezeichnet.

Die Bildung des Erythems ist abhängig von der Wellenlänge. Der Erythembereich des UV-B liegt zwischen 280 nm und 320 nm.

Etwa 90 % der auf die Erde gelangenden ultravioletten Strahlung besteht aus UV-A-Strahlen mit einer Wellenlänge zwischen 320 nm und 400 nm. Während die UV-B-Strahlung in Abhängigkeit von zahlreichen Faktoren stark variiert (z. B. Jahres- und Tageszeit oder Breitengrad), bleibt die UV-A-Strahlung unabhängig von Jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Man hat lange Zeit fälschlicherweise angenommen, daß die langwellige UV-A-Strahlung nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

Da die Beiträge der verschiedenen Wellenlängebereiche des UV-Lichtes zu lichtbedingten Hautveränderungen nicht vollständig geklärt sind, geht man heute verstärkt davon aus, daß vorbeugender Schutz sowohl gegen UV-A- als auch gegen UV-B-Strahlen, beispielsweise durch Auftrag von Lichtschutzfiltersubstanzen in Form einer kosmetischen oder dermatofogischen Formulierung auf die Haut, von grundsätzlicher Wichtigkeit ist. Kosmetische oder dermatologische Mittel sollen, in dünner Schicht auf die Haut aufgetragen, diese vor den negativen Auswirkungen der Sonnenstrahlung schützen.

Ein Sonnenbad wird von den meisten Menschen als angenehm empfunden, die nachteiligen Folgen zunächst nicht beachtet. Allerdings hat sich in den letzten Jahren durchaus ein Bewußtsein über die negativen Auswirkungen einer zu intensiven Sonnenbestrahlung herausgebildet, weshalb mehr und stärker schützende Sonnenschutzmittel angewendet werden.

Weniger Beachtung beim Verbraucher findet hingegen nach wie vor die Tatsache, daß bestimmte Körperpartien - wie beispielsweise das Gesicht oder die Handrücken - der Einwirkung von ultravioletter Strahlung immer dann ausgeliefert sind, wenn man sich im Freien aufhält. Betrachtet man z. B. das Gesicht und die Handrücken und vergleicht dieses Bild mit dem Zustand der Haut an den seitlichen Oberkörperpartien oder mit der Bauchhaut, so erkennt man in der Regel ganz deutlich, daß die üblicherweise bedeckten Hautareale wesentlich jünger wirken.

Selbst die UV-A-Strahlung unter ganz normalen Alltagsbedingungen reicht aus, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Das Alter eines Menschen wird in erster Linie aus der Betrachtung der Gesichtshaut abgelesen, und hier bestehen bei praktisch allen Menschen mehr oder weniger stark ausgeprägte Zeichen einer Lichtschädigung. Für einen konsequenten Sonnenschutz und um eine strahleninduzierte vorzeitige Hautalterung möglichst zu vermeiden, ist es daher sinnvoll, auch Hautpflegeprodukte, deren eigentlicher Zweck nicht der Schutz der Haut vor Sonneneinstrahlung ist, mit Lichtschutzfiltern auszustatten.

Dabei ist allerdings zu beachten, daß beispielsweise an ein Gesichtspflegeprodukt ganz andere kosmetische Anforderungen gestellt werden als an eine Sonnenschutzcreme. Während bei Lichtschutzzubereitungen oft billigend in Kauf genommen werden kann, daß sich bestimmte Bestandteile der Wasserphase - wie z. B. Glycerin - oder der Ölphase - wie z. B. bestimmte UV-Filtersubstanzen - in höheren Konzentrationen negativ auf die sensorischen Eigenschaften der Zubereitungen auswirken, führt ein gesteigertes Klebrigkeits- oder auch Schmierigkeitsgefühl bei der Anwendung als Gesichtscreme im Einzelfall dazu, daß derartige Zubereitungen nicht vermarktungsfähig sein können, da sie vom Verbraucher bzw. der Verbraucherin nicht akzeptiert bzw. negativ beurteilt werden.

Insbesondere wenn hohe Lichtschutzfaktoren (LSF) erreicht werden sollen, kommt häufig noch ein weiteres Problem hinzu. Hohe LSF-Werte sind in der Regel nur durch die Kombination mehrerer Lichtfiltersubstanzen und insbesondere die Verwendung partikulärer (physikalischer) Filter - wie beispielsweise Titandioxid oder Zinkoxid - erhältlich. (Als partikuläre Filter werden im Sinne der vorliegenden Erfindung UV-Filtersubstanzen bezeichnet, deren Filterwirkung im wesentlichen darauf beruht, daß die Sonnenstrahlung reflektiert und/oder gestreut wird.)

Allerdings können Feststoffe bei der Anwendung entsprechender Zubereitungen auf der Haut einen trockenen und zum Teil stumpfen Eindruck hinterlassen. Bereits Zubereitungen mit einem Pigmentgehalt von 1 Gew.-% erzeugen nach ihrer Anwendung ein stumpfes Gefühl auf der Haut, das mit höheren Pigmentkonzentrationen noch zunimmt. Im Einzelfall können daher auch pigmenthaltige Zubereitungen nicht vermarktungsfähig sein.

Aufgabe war daher, den Nachteilen des Standes der Technik Abhilfe zu schaffen. Insbesondere sollten Zubereitungen zur Pflege des Gesichts zur Verfügung gestellt werden, welche auf der Haut weder einen klebrigen bzw. schmierigen noch einen trockenen oder stumpfen Eindruck hinterlassen und welche sich dennoch durch eine hohe UV-Schutzwirkung auszeichnen.

Es war überraschend, daß alle diese Aufgaben gelöst werden durch
kosmetische oder dermatologische O/W-Emulsion zur Pflege der Gesichtshaut, enthaltend
A) mindestens einen Emulgator, gewählt aus der Gruppe der PEG-Ester und/oder PEG-Ether der folgenden Struktur :

   R¹-(CH₂-CH₂-O)ₙ-R²,

   worin R1 und R2 unabhängig voneinander gewählt werden aus der Gruppe : Wasserstoff, Alkyl- und Acylreste mit 12 bis 40 Kohlenstoffatomen und n eine ganze Zahl zwischen 30 und 100 darstellt,
B) mindestens eine UV-Filtersubstanz gewählt aus der Gruppe 1, bestehend aus Phenylen-1,4-bis-(2-benzimidazyl)3,3'-5,5'-tetrasulfonsäure und ihren Salzen
C) mindestens eine UV-Filtersubstanz gewählt aus der Gruppe II, bestehend aus 2-Phenylbenzimidazol-5-sulfonsäure und ihre Salzen und
D) mindestens eine UV-Filtersubstanz gewählt aus der Gruppe III, bestehend aus den Estern der Zimtsäure.

Die Zubereitungen gemäß der vorliegenden Erfindung zeigen sehr gute sensorische und kosmetische Eigenschaften, wie beispielsweise die Verteilbarkeit auf der Haut oder das Einzugsvermögen in die Haut, sie sind weder klebrig noch hinterlassen sie einen stumpfen Eindruck auf der Haut und zeichen sich femer durch eine sehr gute Lichtschutzeffektivität, eine überaus hohe UV-A- und/oder UV-B-Schutzleistung sowie durch hervorragende Hautpflegedaten aus. Es war insbesondere erstaunlich, daß auf übliche partikuläre UV-Filtersubstanzen - wie beispielsweise Titandioxid oder Zinkoxid - gänzlich verzichtet werden kann.

Es ist dementsprechend erfindungsgemäß besonders vorteilhaft, wenn die Zubereitungen deutlich weniger als 0,5 Gew.-% einer oder mehrerer partikulärer UV-Filtersubstanzen enthalten bzw. sogar gänzlich frei von solchen Stoffen sind.

Der oder die Emulgatoren werden erfindungsgemäß vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, . PEG-40-Stearat, PEG-30-Glyceryllaurat, PEG-50-Stearat, PEG-100-Stearat, PEG-150-Laurat, PEG-2 Cetyl/Stearyl Ether, PEG-8 Cetyl/Stearyl Ether, PEG-25 Cetyl/Stearyl Ether, PEG-40 Cetyl/Stearyl Ether, PEG-60, PEG-100 Cetyl/Stearyl Ether, PEG-2 Cetyl Ether, PEG-10 Cetyl Ether, PEG-20 Cetyl Ether, PEG-40 Cetyl Ether, PEG-2 Stearyl Ether, PEG-21 Stearyl Ether, PEG-40 Stearyl Ether, PEG-10 Stearyl Ether . Besonders vorteilhaft im Sinne der vorliegenden Erfindung sind polyethoxylierte Stearinsäureester.

Es ist vorteilhaft im Sinne der vorliegenden Erfindung, die Gesamtmenge der Polyoxyethylenfettsäureester (eine oder mehrere Verbindungen) aus dem Bereich von 0,001 bis 20 Gew.-%, vorteilhaft von 0,002 bis 15 Gew.-%, insbesondere von 0,01 bis 5 Gew.-%, jeweils bezogen auf das Gesamtgewicht der Formulierung, zu wählen.
Erfindungsgemäß vorteilhafte UV-Filtersubstanzen der Gruppen I bis III sind die im folgenden genannten :
- Phenylen-1,4-bis-(2-benzimidazyl)-3,3'-5,5'-tetrasulfonsäure und ihre Salze (Gruppe I):
   Besonders vorteilhaft im Sinne der vorliegenden Erfindung sind die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist.
- 2-Phenylbenzimidazol-5-sulfonsäure und ihre Salze (Gruppe II):
   Besonders vorteilhaft im Sinne der vorliegenden Erfindung sind ihr Natrium-, Kaliumoder ihr Triethanolammonium-Salz sowie die Sulfonsäure selbst mit der INCl Bezeichnung Phenylbenzimidazole Sulfonsäure (CAS.-Nr. 27503-81-7), welche beispielsweise unter der Handelsbezeichnung Eusolex 232 bei Merck oder unter Neo Heliopan Hydro bei Haarmann & Reimer erhältlich ist.
- Ester der Zimtsäure (Gruppe III):
   Besonders vorteilhaft im Sinne der vorliegenden Erfindung sind 4-Methoxyzimtsäure(2-ethylhexyl)ester (2-Ethylhexyl-4-methoxycinnamat, INCl: Ethylhexyl Methoxycinnamate) und 4-Methoxyzimtsäureisopentylester (Isopentyl-4-methoxycinnamat, INCI: Isoamyl p-Methoxycinnamate).

Es ist erfindungsgemäß vorteilhaft, das Gewichtsverhältnis der UV-Filtersubstanzen der Gruppen I bis III wie a : b : c zu wählen, wobei a, b und c unabhängig voneinander rationale Zahlen von 1 bis 5 darstellen können.

Vorteilhaft enthalten die Zubereitungen gemäß der vorliegenden Erfindung die Substanzen der Gruppen I und II in einer Gesamtmenge von jeweils 0,1 Gew.-% bis 15 Gew.-%, vorzugsweise 0,1 bis 5 Gew.-%, insbesondere 0,5 bis 3 Gew.-%, jeweils bezogen auf das Gesamtgewicht der Zubereitungen.

Vorteilhaft enthalten die Zubereitungen gemäß der vorliegenden Erfindung die Substanzen der Gruppe III in einer Gesamtmenge von 0,1 Gew.-% bis 15 Gew.-%, vorzugsweise 0,5 bis 10 Gew.-%, insbesondere 1 bis 5 Gew.-%, jeweils bezogen auf das Gesamtgewicht der Zubereitungen.

Die erfindungsgemäßen kosmetischen oder dermatologischen Lichtschutzformulierungen können wie üblich zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Entsprechend ihrem Aufbau können kosmetische-oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß femer vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u, a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure (Dioic acid, CAS-Nummer 20701-68-2; vorläufige INCl-Bezeichnung Octadecendioic acid).

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination.

Die Zubereitungen gemäß der vorliegenden Erfindung können ferner vorteilhaft auch Selbstbräunungssubstanzen enthalten, wie beispielsweise Dihydroxyacteon und/oder Melaninderivate in Konzentrationen von 1 Gew.-% bis zu 8 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Metadelphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether *(Cetiol OE)* und/oder Dicaprylylcarbonat, beispielsweise das unter der Handelsbezeichnung *Cetiol* CC bei der Fa. Cognis erhältliche.

Es ist ferner bevorzugt, das oder die Ölkomponenten aus der Gruppe Isoeikosan. Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB bei* der Fa. CP Hall erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und lsohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane. welche die mengenmäßig bedeutendsten Verbindungen dieser Gruppe darstellen und sich durch die folgende Strukturformel auszeichnen 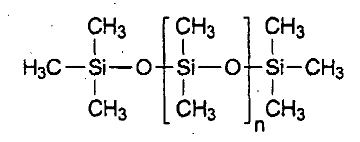 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCl auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem *Rowe Colour Index, 3. Auflage,* Society *of Dyers and Colourists, Bradford, England, 1971* zu wählen.

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'phenyiazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Besonders vorteilhaft sind femer auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Es ist auch vorteilhaft im Sinne der vorliegenden Erfindung, kosmetische und dermatologische Zubereitungen zu erstellen, deren hauptsächlicher Zweck der Schutz vor Sonnenlicht ist.

Dementsprechend können die Zubereitungen im Sinne der vorliegenden Erfindung weitere UV-A-, UV-B- und/oder Breitbandfiltersubstanzen enthalten.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind weitere wasserlösliche UV-Filter, wie z. B.:
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich ;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte weitere UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCl: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoesäure-tris(2-ethylhexylester), auch: 2,4,6-Tris-[anilino-(p-carbo-2'-ethyl-1'-hexyloxy)]-1,3,5-triazin (INCl: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z.B. unter der Handelsbezeichnung Tinosorb® M bei der CIBA-Chemikalien GmbH erhältlich ist.

Vorteilhafter Breitbandfilter im Sinne der vorliegenden Erfindung ist femer das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B. :
- 3-Benzylidencampher-Derivate, vorzugsweise 3-(4-Methylbenzyliden)campher, 3-Benzylidencampher ;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin ;
- Ester der Benzalmalonsäure, vorzugsweise 4-Methoxybenzalmalonsäuredi(2-ethylhexyl)ester ;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

### Vorteilhafte wasserlösliche Filtersubstanzen sind z. B.:

Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 T erhältlich ist.

Die Liste der genannten UV-Filter, die im Sinne der vorliegenden Erfindung als weitere Filtersubstanzen eingesetzt werden können, soll selbstverständlich nicht limitierend sein.

Vorteilhaft enthalten die Zubereitungen gemäß der vorliegenden Erfindung die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

| Beispiele 1.10: O/W-Cremes | | | | | |
|---|---|---|---|---|---|
| **Beispiel Nummer** | **1** | **2** | **3** | **4** | **5** |
| PEG-100-Stearat | 2 | 1 | | | |
| PEG-20-Glycerylstearat | | 1 | | | |
| PEG-40-Stearat | | | | | 1 |
| PEG-25 Cetyl/ Stearyl Ether (Ceteareth-25) | | | 0,5 | | |
| PEG-100Cetyl/ Stearyl Ether (Ceteareth-100) | | | 0,5 | 2 | |
| PEG-40 Cetyl Ether (Ceteth-40) | | | 1 | | |
| Myristylmyristat | 1 | | | | 1 |
| Glyceryl Stearate | | 1 | | | 2 |
| Stearyialkohol | 2 | 1 | | | |
| Cetearylalkohol | | | | 4 | 2 |
| Cetylalkohol | 1 | | 3 | | |
| Hydrierte Kokosfettglyceride (Hydrogenated Coco Glycerides) | 2 | | | | |
| Sheabutter | | 2 | | | 2 |
| C12-15 Alkylbenzoat | | 3 | 2 | | 3 |
| Butylenglycol Dicaprylat/Dicaprat | 1 | | | 1 | |
| Caprylsäure/Caprinsäure Triglycerid | | 1 | 1 | 2 | 2 |
| Ethylhexylkokosfettsäureester | 3 | | | | 1 |
| Octyldodecanol | | | 1 | | |
| Mineralöl | | 1 | | | |
| Vaseline | 2 | | 1 | | 2 |
| Octamethyltetrasiloxan (Cyclomethicon) | 4 | 1 | 4 | 3 | 5 |
| Dimethylpolysiloxan (Dimethicon) | | | 1 | 1 | |
| Dicaprylylether | 1 | 4 | 2 | | |
| Dicarprylylcarbonat | | | | 3 | |
| Ethylhexylmethoxycinnamat | 4 | 3 | 5 | 2 | 2 |
| Bis-lmidazylate | 1 | 1 | 1,5 | 0,5 | 2 |
| Penylbenzimidazole Sulfonic Acid | 2 | 3 | 1 | 2 | 1,5 |
| Ethylhexyltriazon | | | | | 2 |
| Ethylhexylcyanodiphenyl-acrylat (Octocrylen) | | | | 2 | |
| Ethylhexylsalicylat | | | 1 | | |
| Ubichinon (Q10) | 0,05 | | | | |
| Biotin | | | | | 0,04 |
| Retinylpalmitat | | | | 0,1 | |
| α-Liponsäure | 0,1 | | | | |
| Tocopherylacetat | | | 1 | | |
| Citronensäure, Natriumsalz | | 0.1 | | | |
| Natriumascorbylphosphat | 0,1 | | | | 0,1 |
| Trinatrium EDTA | | 0.1 | | | |
| Iminodisuccinat, Natriumsalz | | | 0,1 | | 0,1 |
| Phenoxyethanol | 0,3 | | 0,3 | 0,2 | 0,2 |
| p-Hydroxybenzoesäurealkylester (Paraben) | 0,6 | 0,3 | 0,2 | 0,3 | 0,3 |
| Hexamidindiisethionat | | 0,04 | | | |
| Diazolidinylharnstoff | 0,25 | | 0,1 | 0,2 | 0,1 |
| 1,3-Dimethylol-5,5-dimethyl-hydantoin (DMDM Hydantoin) | | 0,2 | | | |
| lodopropynylbutylcarbamat | | 0,1 | | | |
| Ethanol denaturiert | | 2 | | | |
| Xanthan Gummi | 0,1 | | | | |
| Polyacrylsäure (Carbomer) | 0,05 | | 0,1 | | 0,1 |
| Polyacrylamid | | 0,2 | | | |
| Glycerin | 10 | 6 | 6 | 7,5 | 8 |
| Butylenglycol | 2 | 1 | | | |
| wasser- und/oder öllösliche Farbstoffe | 0,05 | | | | |
| Füllstoffe/ Additive (Distärke-phosphat, SiO₂, BHT, Talkum, Aluminiumstearat) | 0,1 | 1 | 0,2 | 0,5 | 0,05 |
| Parfüm | q.s. | q.s. | q.s. | q.s. | q.s. |
| Wasser | Ad 100 | Ad 100 | Ad 100 | Ad 100 | Ad 100 |

| Beispiel Nummer | 6 | 7 | 8 | 9 | 10 |
|---|---|---|---|---|---|
| PEG-50-Stearat | 2,5 | | | | 1 |
| PEG-40-Stearat | 1 | 1 | | | 0,5 |
| PEG-8-Stearat | | | | 1 | |
| PEG-8-Distearat | | | | | 1 |
| Glyceryl Stearate | | 3 | | | |
| Sorbitanstearat | | 1 | | | |
| Polyethylenglycol(21 )stearyl-ether (Steareth-21 ) | | | 2 | 1 | |
| Polyethylenglycol(2)stearyl-ether (Steareth-2) | | | 1 | | |
| Cetearylglucosid | | | | 2 | |
| Myristylmyristat | | | | 1 | |
| Behenylalkohol | | 1 | | | 2 |
| Stearylalkohol | | | | 5 | |
| Cetearylalkohol | 3 | | 2 | | 1 |
| Cetylalkohol | | 1 | | | |
| Hydrierte Kokosfettglyceride (Hydrogenated Coco Glycerides) | 1 | | | | 1 |
| Sheabutter | 2 | | | | |
| C12-15 Alkylbenzoat | | 2 | 5 | 2 | |
| Butylenglycol Dicaprylat/Dicaprat | 1 | | | | 2 |
| Caprylsäure/Caprinsäure Triglycerid | 1 | 1 | | 3 | |
| Hydriertes Polydecen | | | | 1 | |
| Ethylhexylkokosfettsäureester | | | | | 2 |
| Octyldodecanol | | | 1 | | 1 |
| Mineralöl | | | 1 | | |
| Vaseline | 1 | | | | |
| Octamethyltetrasiloxan (Cyclomethicon) | 4 | 3 | 2 | | 2 |
| Dimethylpolysiloxan (Dimethicon) | | | | | 1 |
| Dicaprylylether | | | 2 | | |
| Dicarprylylcarbonat | | 2 | | 3 | 4 |
| Polydecen | | | | 4 | |
| Ethylhexylmethoxycinnamat | 2 | 3 | 4 | 5 | 4 |
| Phenylbenzimidazol-sulfonsäure | 0,5 | 2 | 2 | 3 | 1 |
| Bis-lmidazylate | 1 | 1 | 1,5 | 2 | 0,5 |
| Ubichinon (Q10) | 0,03 | | | | |
| Biotin | | 0,02 | | | |
| Retinylpalmitat | | | | 0,2 | |
| Tocopherylacetat | | 1 | | | 0,5 |
| Ascorbinsäure | | | 0,05 | | |
| Lactoferrin | | | | | 0,05 |
| Trinatrium EDTA | | | 0,2 | 0,1 | |
| Iminodisuccinat | | 0,2 | | | 0,1 |
| Phenoxyethanol | 0,5 | 0,4 | 0,5 | | 0,3 |
| p-Hydroxybenzoesäurealkylester (Paraben) | 0,1 | | | 0,4 | 0,6 |
| Hexamidindiisethionat | | | 0,1 | | |
| Diazolidinylharnstoff | 0,2 | 0,2 | | 0,1 | |
| lodopropynylbutylcarbamat | | | 0,25 | | |
| Ethanol denaturiert | | 8 | | | 3 |
| 2-Ethylhexylglycerinether (Octoxyglycerin) | | | | 0,4 | |
| Xanthan Gummi | | 0,1 | | | |
| Polyacrylsäure (Carbomer) | 0,2 | | 0,1 | | 0,1 |
| Polyacrylamid | | 0,2 | | | |
| Glycerin | 10 | 5 | 6 | 4 | 7 |
| Butylenglycol | | | | 2 | |
| wasser- und/oder öllösliche Farbstoffe | | | | | 0,1 |
| Additive (Distärkephosphat, SiO₂, Talkum, BHT Aluminiumstearat) | 0.03 | | 0,05 | 3 | |
| Parfüm | q.s. | q.s. | q.s. | q.s. | q.s. |
| Wasser | Ad 100 | Ad 100 | Ad 100 | Ad 100 | Ad 100 |

## Patentansprüche

1. Kosmetische oder dermatologische O/W-Emulsionen zur Pflege der Gesichtshaut, enthaltend
A) mindestens einen Emulgator, gewählt aus der Gruppe der PEG-Ester und/oder PEG-Ether der folgenden Struktur:
R¹-(CH₂-CH₂-O)ₙ-R²,
worin R1 und R2 unabhängig voneinander gewählt werden aus der Gruppe: Wasserstoff, Alkyl- und Acylreste mit 12 bis 40 Kohlenstoffatomen und n eine ganze Zahl zwischen 30 und 100 darstellt,
B) mindestens eine UV-Filtersubstanz gewählt aus der Gruppe l, bestehend-aus Phenylen-1,4-bis-(2-benzimidazyl)-3,3'-5,5'-tetrasulfonsaure und ihren Salzen
C) mindestens eine UV-Filtersubstanz gewählt aus der Gruppe II, bestehend aus 2-Phenylbenzimidazol-5-sulfonsäure und ihre Salzen und
D) mindestens eine UV-Filtersubstanz gewählt aus der Gruppe III, bestehend aus den Estern der Zimtsäure.

2. Emulsionen nach Anspruch 1, **dadurch gekennzeichnet, daß** sie weniger als 0,5 Gew.-% einer oder mehrerer partikulärer UV-Filtersubstanzen enthalten.

3. Emulsionen nach einem der Ansprüche 1 oder 2, **dadurch gekennzeichnet, daß** sie frei von partikulären UV-Filtersubstanzen sind.

4. Emulsionen nach einem der vorhergehenden Ansprüche, **dadurch gekennzeichnet, daß** als Emulgator PEG-40 Stearat gewählt wird.

5. Emulsionen nach einem der vorhergehenden Ansprüche, **dadurch gekennzeichnet, daß** als UV-Filtersubstanzen aus der Gruppe I das Phenylen-1,4-bis-(2-benzimidazyl)-3,3'-5,5'-tetrasulfonsäure-bis-natriumsalz, aus der Gruppe II die Phenylbenzimidazole Sulfonsäure und aus der Gruppe III der 4-Methoxyzimtsäure(2-ethylhexyl)ester gewählt werden.

6. Verwendung von Emulsionen nach einem der vorhergehenden Ansprüche zur Pflege der Gesichtshaut.
